(19) Europäisches Patentamt
European Patent Office
Office européen des brevets

(11) Publication number: 0 261 351 A2

(12) **EUROPEAN PATENT APPLICATION**

(21) Application number: 87111100.1

(51) Int. Cl.4: A61K 7/46

(22) Date of filing: 31.07.87

(30) Priority: 26.09.86 EP 86113250

(43) Date of publication of application: 30.03.88 Bulletin 88/13

(84) Designated Contracting States: CH DE ES FR GB IT LI NL

(71) Applicant: Roure Bertrand Dupont Société Anonyme
55, Voie des Bans
F-95102 Argenteuil(FR)

(72) Inventor: Blakeway, John Murray
3, route de Magny
F-95420 Nucourt(FR)

(74) Representative: Urech, Peter, Dr. et al
Grenzacherstrasse 124 Postfach 3255
CH-4002 Basel(CH)

# (54) Stable, aqueous or aqueous-alcoholic solutions of perfume oils.

(57) The invention relates to stable. aqueous or aqueous-alcoholic-solutions of fat soluble perfume oils, containing,

a) a $C_{1-6}$-alkanol propylene oxide and ethylene oxide polymer (oligomer) and
b) a non-ionic emulsifier.

The invention relates also to the preparation of these novel solutions, to the solubilising agents being useful to prepare the solutions captioned above, to the method of solubilizing fat soluble perfume oils using the novel solubilizing agents, and to the use of the novel solutions.



Xerox Copy Centre

## Stable, aqueous or aqueous-alcoholic solutions of perfume oils

The invention relates to stable, aqueous or aqueous-alcoholic solutions of fat soluble perfume oils, containing,

a) a $C_{1-6}$-alkanol propylene oxide and ethylene oxide polymer (oligomer) and

b) a non-ionic emulsifier.

The invention relates also to the preparation of these novel solutions, to the solubilising agents being useful to prepare the solutions captioned above, to the method of solubilizing fat soluble perfume oils using the novel solubilizing agents, and to the use of the novel solutions.

The term $C_{1-6}$-alkanol should embrace straight and branched alkanols; examples of such alkanols are methanol, ethanol, propanol, i-propanol, n-butanol (preferred), sec-butanol, i-butanol, amyl alcohol, hexanol.

The nature of the non-ionic emulsifier is not critical and practically all these emulsifiers may be used for the purpose of the present invention.

As non-ionic emulsifiers there are, however, preferably used ethoxylated or propoxylated/ethoxylated derivatives of:

aliphatic alcohols, in particular $C_{12-20}$ aliphatic alcohols,
alkylphenols,
aliphatic carboxylic acid (fatty acid) esters,
fatty acid esters of anhydrosorbitol,
natural fats, oils and waxes.

All the various suitable types of non-ionic emulsifiers are well-known in the art, see e.g. the respective entries in the appropriate encyclopediae of, e.g. Ullmann, Kirk-Othmer and Römpp, as well as the appropriate cosmetic standard works, such as those from Janistyn, Nowak, Balsam/Sagarin, Shinoda, or the CFTA Cosmetic Ingredient Dictionary and the various leaflets provided by the suppliers listed in the literature.

The term ($C_{12-20}$-)aliphatic alcohols should embrace saturated and ethylenically unsaturated straight and branched, native and synthetic fatty alcohols. Examples are: cetyl, (iso) stearyl, cocoyl (i.e. fatty alcohols resulting from the hydrogenation of coconut oil), lauryl, oleyl, myristyl, capryl, tridecyl, eicosyl alcohols.

The preferred alcohols are the $C_{16-18}$-aliphatic alcohols, e.g. cetyl, stearyl and oleyl alcohol.

The term alkylphenol should particularly embrace mono-, di-and tri-$C_{8-15}$ alkyl-phenols, whereby the alkyl radicals can be straight or branched. Examples are octyl, nonyl, dodecyl; octyl and nonyl are preferred.

The term aliphatic carboxylic acid (fatty acid) esters should embrace monoesters, diesters, sesquiesters of the fatty acids, e.g. of $C_{12-18}$-acids, particularly $C_{16-18}$-acids; examples of such acids are lauric, (iso) stearic, myristic, palmitic, stearic (preferred), linolenic linoleic, oleic, coco (i.e. fatty acids derived from coconut oil), ricinoleic, castor, etc. and hydrogenated castor oil fatty acids (preferred).

The term relates primarily to the respective polyoxyethylene (preferred) and polyoxypropylene/polyoxyethylene esters of the fatty acids enumerated above.

The term should, however, also embrace esters with polyhydroxy compounds, e.g. ethylene glycol, polyethylene glycol, glycerol, diglycerol, polyglycerol, propylene glycol, sorbitol, etc.

The term fatty acid esters of anhydrosorbitol relates to mono-, di-and triesters of sorbitan and fatty acids, e.g. to the oleates (preferred), to the laurates, to the palmitates, the stearates, etc.

The term natural fats, oils and waxes relates preferably to such compounds as castor oil (triglyceride high in ricinoleic esters), to lanolin, to beeswax, etc.

The average number of ethylene oxide units (degree of ethoxylation, EO) in the propoxylated and ethoxylated component a) is suitably between 10 and 40, preferably between 25 and 35; the respective number of propylene oxide units (degree of propoxylation, PO) is also suitably between 10 and 40, also preferably between 25 and 35.

The terms "ethoxylated" and "propoxylated-ethoxylated" relate to the condensation products of ethylene oxide and/or propylene oxide with substrates having acid (movable) protons, i.e. those enumerated above for component b).

In an analogous manner, the term "propylene oxide and ethylene oxide polymer" relates to the respective condensation products of the group of compounds outlined above for a) with ethylene oxide and propylene oxide.

e.g. $C_{1-6}$-alkanol + $H_2C-CH_2$ (epoxide, $\backslash O /$) condense reaction product with

$H_2C-CH-CH_3$ (epoxide, $\backslash O /$) (in case of a)), and

$C_{12-20}$-aliphatic alcohol + $H_2C-CH_2$ (epoxide, $\backslash O /$), or

e.g. $C_{12-20}$-aliphatic alcohol + $H_2C-CH_2$ (epoxide, $\backslash O /$), condense reaction product

with $H_2C-CH-CH_3$ (epoxide, $\backslash O /$) (in case of b))

The reaction involved is generically an alkylation (i.e. an oxalkylation, a hydroxethylation), the products resulting from this reaction are thus usually termed ethoxylates or oxethylates and propoxylates or oxpropylates.

The respective figures for EO in the components b) are suitably between 8 and 200, preferably between 30 and 50, PO is suitably 0 to 20, preferably 0 to 6, most preferably 2 to 4.

A preferred class of $C_{1-6}$-alkanol derivatives are butanol derivatives, e.g. as represented by formula

$$C_4H_9(O-C_3H_6)_aO(C_2H_4)_bOH \qquad II$$

wherein a = 20 to 40, preferably 20 to 30 and b = 20 to 40, Preferably 25 to 35.

If a = b = 26, the product involved is Witconol APEB (Witco Chemical, Organics Div.).

In all cases where a number of ethylene oxide or propylene oxide groups is given (example : butyl alcohol 26 PO 26 EO), this is an average number or molecules added. It is well-known that this number represents a statistical average, and in a given composition, the proportion of molecules present with the number shown will very often not be greater than 30 to 40% of the total number of molecules.

Fat soluble perfume oils are natural or synthetic essential oils, such as orange oil, pine oil, peppermint oil, eucalyptus oil, lemon oil, clove leaf oil, cedarwood oil, bergamot oil, rosmary oil, patchouli oil, lavandin oil, spike oil, rose oil, vetiver oil, fennel oil, anise oil, thyme oil, geranium oil, lavender oil, menthol and synthetic oil soluble perfume oils, preferably selected from the group consisting of aldehydes, esters and polyenic compounds, etc. Naturally the term also encompasses any mixtures of perfume oil as defined herein.

The usual procedure for solubilizing perfume oils into, e.g. o/w-solutions is to mix these perfume oils with the solubilizing agents, conveniently in the ratio of ca. 3 to 1, preferably ca. 2 to 1 with the solubilizing agents, and, optionally the further oleophilic products, e.g. preservatives, emollients, e.g. lanolin, further solvents, colours, etc. destined for the final formula. The water, conveniently in an amount of ca. 50 to 99 weight%, preferably of ca. 80 to 90 weight% is then added in small portions, at first with vigorous mixing ensuring that such addition is well absorbed by the perfume solubiliser mixture (leading to an opalescent or even clear solution) before the next addition.

Towards the end of the addition the water may be added in larger amounts, e.g. the last 50% of the water may be added in one addition.

In some cases the initial perfume/solubilizer/water mixture may give a gel which requires mixing with greater care than usual, until with further additions of the water the mixture becomes liquid.

The temperature may vary from 0°C to 100°C although temperatures above 50°C are better avoided due to the insolubility of many nonionic solubilisers at higher temperatures.

The main advantage of the novel solubilizing agents consists in the fact that these agents can be used in considerably lower and thus more economical concentrations. Usually a range of ratios of perfume: solubilizer of 0.3 to 1.4, preferably a range of 0,5 to 1,0 parts of the novel mixture M is sufficient to solubilize one part of perfume. Moreover, the novel solubilizing agents make it possible to use smaller quantities of alcohol to prepare the desired final solutions.

Mixtures of different type a) and type b) components may be used in the same formulation to obtain improved solubility of fragrances.

The novel solubilized perfume oil solutions may be used in any type of cosmetic formulations, e.g. in (aqueous or aqueous alcoholic) solutions, in lotions, tonics, fixatives, sprays, emulsions, gels, oils, creams, salves, sticks, conditioners, cleaners; a preferred use is that in room deodorants.

Suitable and preferred parameters are as follows:

| | convenient | preferred |
|---|---|---|
| amount of perfume oil in stabilized solution | 0,05% to 25% | 0,1% to 10% |
| amount of a) + b)/perfume oil | 10% to 140% | 25% to 100% |
| amount of a) + b) in stabilized solutions | 0.01% to 40% | 0,05% to 10% |
| [$H_2O$] in stabilized solution | 35% to 99.94% | 70% to 99% |
| [$C_2H_5OH$]* in stabilized solutions | <30% | 0% to 20% |
| amount of stabilized solution in cosmetic formulation | 50% to 100% | 70% to 100% |
| amount of stabilized solution in room deodorant | ~100% | ~95% |

* or other equivalent solvent, such as another lower alcohol, e.g. a lower alkanol, e.g. methanol, n- or iso-propanol, etc., or the mono-ethylene, di-ethylene, propylene, dipropylene or butylene glycol ethers of these alkanols, etc.

The standard works listed above give, furthermore, all the necessary information of how to prepare the various types of the final cosmetic formulations. Reference is also made to Examples a) to r) below.

We believe that the solubilizer is the surfactant acting as an agent to produce visually clear or opalescent solutions (or micro-emulsions), whereas the emulsifier is the agent which produces the disperse system of the two non-miscible fluids in more or less fine distribution, in other words, the agent being necessary to emulsify the perfume oil into an aqueous or aqueous/alcoholic solution, such agent causing the perfume oil to form droplets which are uniformly distributed throughout the solution.

The term solubilizer, in the context of the present invention, is, however, for convenience sake often used to indicate the total of the two components a) and b).

We have the theory that component a) is primarily acting as the solubilizer, and component b) is acting primarily as the emulsifier.

It is well-known in the art that aqueous systems containing surfactants and perfumes are susceptible to microbial contamination and for the products to be useful in long term storage, suitable preservatives should therefore be added.

The nature of these preservatives depends on the end use of the product. If it is not to enter in contact with human skin, 0.05% to 0.15% formaldehyde may be used, whilst if the product is designed for human use, a mixture such as:

-0,1% methyl parahydroxybenzoate,
-0,15% propyl parahydroxybenzoate
-0,2% N,N''-methylenebis
(N'-(1-hydroxymethyl)-2,5-dioxo-4-imidazolidinyl) urea) (e.g. Germall 115 - Sutton Laboratories Inc.) may be used. If greater than 20% alcohol is included in the formula, no additional preservative is usually required.

## Examples

a) Perfume solution for room fresheners

| | parts(per weight) |
|---|---|
| perfume base "woody rose" | 4 |
| butyl alcohol (29 PO, 29 EO): stearyl alcohol (20 EO, e.g. BRIJ 78-ICI Americas) = 60:40 | 3 |
| water, colour, preservative | 93 |
| | 100 |

The ethoxylates are melted and mixed at 40°, the perfume base is added and the water is added slowly with mixing to give a clear solution.

b) Perfume solution for window cleaners

| | | parts |
|---|---|---|
| perfume base "pine" | | 0.15 |
| butyl alcohol (29 PO, 29 EO) | | 0.03 |
| polyethyleneglycol monostearate (400 EO, e.g. MYRJ 45-ICI Americas) | | 0.02 |
| dipropylene glycol monomethyl ether | | 6.5 |
| | | 6.7 |
| water | to | 100 |

c) Perfume solution for cleaning the body

| | parts |
|---|---|
| perfume base "lilac hyacinth blend with musky fixatives" | 3.5 |
| butyl alcohol (29 PO, 29 EO) | 1.3 |
| cetostearyl alcohol (mixture of cetyl alcohol and stearyl alcohol) (100 EO) | 0.7 |
| ethanol | 20 |
| water | 74.5 |
| | 100 |

d) Hair setting gel

| | parts |
|---|---|
| perfume base as above | 0.5 |
| butyl alcohol derivative as above | 0.2 |
| cetostearyl alcohol as above | 0.2 |
| Carbopol 941 (crosslinked polyacrylic acid (B.F. Goodrich) | 0.25 |
| PVP/V A 735 (polyvinyl acetate/ vinyl pyrrolidone copolymer; hair setting resin) | 2.4 |
| ethanol | 20 |
| diisopropanolamine ad pH 7.01 | |
| water | to 100 |

e) Perfume solution for room deodorants

| | parts |
|---|---|
| perfume base "rose" | 5 |
| butyl alcohol (29 PO, 29 EO) | 2.4 |
| stearyl alcohol (3 PO, 20 EO) | 1.6 |
| water | 91 |
| | 100 |

f) Perfume solutions for room deodorants

A series of compositions was made up and the intensity of their odours assessed on a scale of 0 = odourless to 5 = strong.

A. solubilizer

| | mixture number A | B | C | D |
|---|---|---|---|---|
| butyl alcohol (29 PO, 29 EO) | 60 | 60 | 50 | 60 |
| stearyl alcohol (20 EO) | 40 | 30 | | |
| stearyl alcohol (100 EO, e.g. EMPILAN KM 100 (Marchon)) | | 10 | | |
| cetyl alcohol (20 EO, e.g. BRIJ 58-ICI Americas) | | | 50 | |
| polyethylene glycol monostearate (400 EO) | | | | 40 |

B. novel perfume solution

| Composition reference | Perfume base | Perfume quantity | Mixture quantity mixture reference A | B | C | D | water |
|---|---|---|---|---|---|---|---|
| 21A | Violet R 921 | 4.0 | 3.5 | | | | ad 100 |
| 22A | Amber RD4568 | 10.0 | | 10.0 | | | |
| 23A | Lemon RD 3511 | 6.0 | | | 5.0 | | |
| 24A | Musk R 305 | 3.0 | | | | 2.5 | |

Violet R 921:

Floral bouquet dominated by a violet base note.

Amber RD 4568:

Woody fern note with warm amber overtones.

Lemon RD 3511:

Citrus cologne based on bergamot and lemon oils.

Musk R 305:

Oriental spicy blend with strong residual musk undertones.
Water was added in each case to a total quantity of 100.

D. Traditional compositions

| Composition | Ethanol | hydrogenated castor oil (60 EO) | water |
|---|---|---|---|
| 21B | 30 | 8.0 | |
| 22B | 50 | 12.0 | ad 100 |
| 23B | 25 | 5.0 | |
| 24B | 30 | 7.0 | |

Water was added in each case to a total quantity of 100.
The odour intensity was compared at the same conditions, i.e. at the same evaporation rate on a rating scale of 0 to 5 (0 odourless, 5 = strong). The figures listed are the mean of 10 trained observers.

| | | | |
|---|---|---|---|
| 21A | 3.6 | 21B | 2.8 |
| 22A | 4.4 | 22B | 3.7 |
| 23A | 4.1 | 23B | 3.3 |
| 24A | 3.8 | 24B | 2.9 |

These results show the improvement in odour intensity with the novel solubilizers compared with a conventional system of solubilisation.

g) Solubilizer efficacy

Solubilizer mixture required to solubilize various amounts of perfume bases in water:

| A | Parts | | | | |
|---|---|---|---|---|---|
| Perfume Oeillet RD 9181 | 1.0 | 3.0 | 4.0 | 5.0 | 10.0 |
| solubilizer base number 1 | 0.9 | 2.0 | 3.5 | 4.0 | 9.0 |
| water | to 100 parts | | | | |

Perfume Oeillet RD 9181:

Floral fragrance with carnation hyacinth top notes.

| Base number 1 | parts |
|---|---|
| butanol (29 PO, 29 EO) | 60 |
| stearyl alcohol (3 PO, 10 EO) | 40 |

| B | Parts | | |
|---|---|---|---|
| Perfume Strawberry RD 9182 | 5 | 5 | 5 |
| Base SSII | 3.5 | 3 | 4 |
| ethanol (co-solvent) | 10 | 20 | 0 |
| water | to 100 parts | | |

Perfume Strawberry RD 9182:

Fresh fruity aroma with dominant strawberry note.

| Base SSII | parts |
|---|---|
| butanol (29 PO, 29 EO) | 55 |
| stearyl alcohol (3 PO, 10 EO) | 35 |
| stearyl alcohol (100 EO) | 10 |
| | 100 |

h) Antiperspirant formula for use in roll-on applications

| | Parts |
|---|---|
| aluminium chlorhydroxide (50% aqueous solution) | 20 |
| ethyl alcohol (20 PO, 25 EO) | 0,8 |
| lauryl alcohol (6 PO, 50 EO) | 0,4 |
| bouquet "carnation" | 1,5 |
| water, colour | to 100 |

The ethoxylates and perfume are melted and mixed at 40° and the water added slowly with mixing to give a clear solution. The finished anti-perspirant formula is completed by the addition of the aluminium chlorhydroxide solution and coloured.

i) Baby perfume

| | Parts |
|---|---|
| cologne fragrance | 2,0 |
| monooleate ester of anhydrosorbitol ethoxylate (20 EO) | 1,3 |
| ethyl alcohol (20 PO, 25 EO) | 0,6 |
| water, preservatives | to 100 |

The ethoxylates and perfume are melted and mixed at 30° and diluted with the water. The preservatives are then added. 20% of the water in this formula may be replaced by ethanol which is added to the mixture of fragrance and ethoxylates before the water addition.

j) Household surface cleaner

| | Parts |
|---|---|
| sodium pyrophosphate | 5 |
| nonyl phenol (5 PO, 20 EO) | 0,45 |
| methyl alcohol (35 PO, 30 EO) | 0,55 |
| perfume "pine" | 1,1 |
| water, preservative | to 100 |

The perfume ethoxylate mixture is diluted with 20 parts of the water. The sodium pyrophosphate is dissolved in the remaining water which is then added to the perfume solution. Other surfactants and hydrotropes such as sodium xylene sulphonate, etc. may also be added to the formula if required.

k) Hair tonic

| | Parts |
|---|---|
| vitamin A palmitate | 0,1 |
| panthenol | 0,1 |
| lanolin alcohol (15 PO, 25 EO) | 0,3 |
| butyl alcohol (25 PO, 25 EO) | 0,2 |
| perfume (mentholated eucalyptus) | 0,5 |
| lauryl isoquinolinium bromide | 0,5 |
| isopropyl alcohol | 6,0 |
| water | to 100 |

All the ingredients, except the water, are dissolved in the isopropyl alcohol. The water is then added slowly to obtain a clear solution.

l) Hair conditioner

| | Parts |
|---|---|
| dimethyl siloxane/ethylene glycol/copolymer | 1,0 |
| dilauryl dimethyl ammonium chloride | 1,5 |
| alcohol | 15,0 |
| hexyl alcohol (20 PO, 30 EO) | 0,5 |
| glyceryl monostearate (40 EO) | 0,9 |
| perfume | 1,0 |
| water, preservatives, colour | to 100 |

For the procedure see example k).

m) Permanent wave solution

| | Parts |
|---|---|
| ammonium thioglycolate | 8,0 |
| alcohol | 16,0 |
| hexyl alcohol (20 PO, 30 EO) | 0,5 |
| glyceryl monostearate (40 EO) | 0,9 |
| hyacinth perfume | 1,0 |
| water colour | to 100 |

The similar procedure to that used for example k) is used for the permanent wave solution, precautions being taken to avoid ammonia loss.

n) Hair setting lotion

| | Parts |
|---|---|
| polyvinylpyrolidone (M.W. ~10000) | 1,5 |
| polyacrylic acid | 1,0 |
| 2-amino-2-methyl-propanol | 0,24 |
| perfume "paeony" | 0,5 |
| hydrogenated castor oil (100 EO, e.g. NIKKOL HCO-100 (Nikko)) | 0,4 |
| butyl alcohol (25 PO, 25 EO) | 0,2 |
| water, preservatives | to 100 |

The polyacrylic acid is dispersed in 50 parts of water. The perfume and ethoxylates are blended together and diluted with the remaining water to which is then added the amino methyl propanol and polyvinylpyrolidone. The mixture is then thickened by the addition of the polyacrylic acid solution.

o) Mouthwash

| | Parts |
|---|---|
| alcohol | 12,5 |
| glycerin | 4,5 |
| sodium lauryl sulphate | 0,3 |
| saccharin | 0,06 |
| mandarin oil | 0,2 |
| monooleate ester of anhydrosorbitol ethoxylate (20 EO, e.g. TWEEN 80-ICI Americas) | 0,21 |
| butyl alcohol (29 PO, 29 EO) | 0,09 |
| water | 82,14 |

The ingredients other than water are dissolved in the alcohol and water is slowly added with stirring to give a solubilised flavour.

p) WC deodorant solution

| | | parts |
|---|---|---|
| butyl alcohol (26 EO, 26 PO) (e.g. Witconol APEB - WITCO) | | 1,5 |
| hydrogenated castor oil (40 EO) (e.g. Cremophor RH 40 - BASF) | | 3,5 |
| perfume pine forest | | 5,0 |
| water and preservative | to | 100 |

q) Kitchen deodorant solution (1)

| | | parts |
|---|---|---|
| butyl alcohol (26 EO, 26 PO) (e.g. Witconol APEB - WITCO) | | 4,0 |
| stearyl alcohol (9PO, 3EO) (e.g. Witconol APES - WITCO) | | 3,5 |
| citron aroma | | 6,5 |
| water and preservatives | to | 100 |

r) Kitchen deodorant solution (2)

| | | parts |
|---|---|---|
| butyl alcohol (26 EO, 26 PO) (e.g. Witconol APEB - WITCO) | | 1,5 |
| stearyl alcohol (9 PO, 3 EO) (e.g. Witconol APES - WITCO) | | 4,5 |
| lanolin (12 PO, 50 EO) (e.g. Laurexol AWS - CRODA) | | 0,5 |
| bergamot oil | | 6,5 |
| water and preservatives | to | 100 |

In each case p, q and r respectively, the making procedure is to mix the melted solubilisers with the perfume, adding, if necessary, further perfume and/or aroma and then slowly add the water with vigorous mixing to ensure that each addition is thoroughly blended before adding subsequent portions.

## Claims

1. A stable, aqueous or aqueous-alcoholic solution of fat soluble perfume oils, containing
   a) a $C_{1-6}$-alkanol propylene oxide and ethylene oxide polymer (oligomer) and
   b) a non-ionic emulsifier.

2. A solution according to claim 1, wherein n-butyl alcohol is the $C_{1-6}$-alkanol of component a).

3. A solution according to claim 1 or 2, wherein the number of ethylene oxide units (EO) in the propoxylated/ethoxylated polymer (oligomer) is 10 to 40, preferably 25 to 35, and the number of propylene oxide units (PO) is 10 to 40, preferably 25 to 35.

4. A solution according to anyone of claims 1 to 3, wherein the emulsifier is selected from the group of surfactants consisting of ethoxylated or propoxylated/ethoxylated
   aliphatic alcohols
   alkylphenols
   aliphatic carboxylic acid (fatty acids) esters
   fatty acid esters of anhydrosorbitol
   natural fats, oils and waxes.

5. A solution according to claim 4, wherein the number of ethylene oxide units in the ethoxylated and/or propoxylated and ethoxylated derivative b) is 8 to 200, preferably 30 to 50, the number of propylene oxide units is 0 to 20, preferably 0 to 6, most preferably 2 to 4, the aliphatic alcohol contains from 12 to 20 carbon atoms, preferably from 16 to 18 carbon atoms, the fatty acid is a $C_{12\ to\ 18}$, preferably a $C_{16\ to\ 18}$ alcanoic or alcenoic acid, and the alkyl moiety of the phenyl exhibits 8 to 15, preferably 8 or 9 carbon atoms.

6. A solution according to claim 5, wherein the solubilizing agent b) is stearyl alcohol (0 to 20 PO and 10 to 100 EO).

7. A solution according to claim 2, wherein the solubilizing agent b) is cetyl alcohol (0 to 15 PO and 10 to 100 EO).

8. A solution according to claim 2, wherein the solubilizing agent b) is a mixture of cetyl and stearyl alcohol (0 to 20 PO and 10 to 80 EO).

9. A solution according to claim 2, wherein the solubilizing agent b) is nonyl phenol (0 to 10 PO and 20 to 100 EO).

10. A solution according to claim 2, wherein the solubilizing agent b) is oleyl alcohol (0 to 8 PO and 15 to 80 EO).

11. A solution according to claim 2, wherein the solubilizing agent b) is sorbitan monooleate (20 to 60 EO).

12. A solution according to anyone of claims 1 to 11, wherein the butyl alcohol-ethylene oxide and/or propylene oxide Polymer (oligomer) is of the formula

$C_4H_9(O\text{-}C_3H_6)_aOH(OC_2H_4)_bOH$ II

wherein a = 20 to 40, preferably 20 to 30 and b = 20 to 40, preferably 25 to 35.

13. A solution according to claim 9, wherein the butyl alcohol-ethylene oxide and propylene oxide polymer (oligomer) is the butyl alcohol (25 to 30 PO and 25 to 30 EO).

14. A solution according to any one of claims 1 to 13, wherein the ratio of the component a) to component b) is from 10:95 to 90:5, preferably from 20 - 80 to 80 - 20.

15. A solution according to any one of claims 1 to 14, wherein the perfume oil is present in an amount of ca. 0,05 to ca. 25 weight%, preferably of ca. 0,1 to ca. 10 weight%, of the total mixture.

16. A solution according to any one of claims 1 to 15, wherein the components a) and b) are present in amounts of ca. 0,01 to ca. 40 weight%, preferably of ca. 0,05 to ca. 10 weight %, of the total solution.

17. A solution according to any one of claims 1 to 16, wherein components a) and b) are present in an amount of ca. 10 to ca. 140 weight%, preferably of ca. 25 to ca. 100 weight%, of the perfume oil.

18. A solution according to any one of claims 1 to 17, wherein the water content is ca. 35 to ca. 99,94 weight%, preferably ca. 70% to ca. 99% weight%.

19. A solution according to any one of claims 1 to 17, wherein the ethanol content is 10 to 30 weight%, preferably ca. 0 to ca. 20 weight%.

20. A process for the preparation of the solution of any one of claims 1 to 19, comprising mixing the perfume oil with components a) and b), and, if necessary, with further organoleptic and/or auxiliary materials, and adding the water under stirring.

21. Use of the mixture of components a) and b) as defined in any one of claims 1 to 5 or of the solutions of any one of claims 1 to 19 in cosmetic formulations, preferably in room deodorants.

22. Method for the solubilization of perfume oils, comprising using a mixture of components a) and b) as defined in any one of claims 1 to 5.

23. Mixtures of components a) and b) as defined in any one of claims 1) to 19).

24. A cosmetic formulation, containing a mixture of components a) and b) as defined in claim 23, preferably in the form of a solution as defined in any one of claims 1 to 19.

25. A room deodorant, containing a mixture of components a) and b) as defined in claim 23, preferably in the form of a solution as defined in anyone of claims 1 to 19.